**Europäisches Patentamt**

**European Patent Office**

**Office européen des brevets**

(19)

(11) **EP 1 417 927 A1**

(12) **EUROPÄISCHE PATENTANMELDUNG**

(43) Veröffentlichungstag:
   **12.05.2004  Patentblatt 2004/20**

(51) Int Cl.⁷: **A61B 5/0428**, A61B 5/055,
   A61B 5/024

(21) Anmeldenummer: **02405968.5**

(22) Anmeldetag: **11.11.2002**

(84) Benannte Vertragsstaaten:
   **AT BE BG CH CY CZ DE DK EE ES FI FR GB GR
   IE IT LI LU MC NL PT SE SK TR**
   Benannte Erstreckungsstaaten:
   **AL LT LV MK RO SI**

(71) Anmelder: **Schiller AG
   6340 Baar (CH)**

(72) Erfinder:
   • **Schmid, Johann-Jakob
   8911 Rifferswil (CH)**

   • **Abächerli, Roge
   6312 Steinhausen (CH)**
   • **Felblinger, Jacques
   54710 Ludres (FR)**

(74) Vertreter: **Müller, Christoph Emanuel et al
   Hepp, Wenger & Ryffel AG,
   Friedtalweg 5
   9500 Wil (CH)**

(54) **Verfahren und Vorrichtung zum Erfassen und Übermitteln von elektrophysiologischen Signalen zur Verwendung in einem MRI-System**

(57)   Zum Übertragen von elektrophysiologischen Daten aus einem Störungsbereich (S) einer MRI Anordnung (20) zu einer Auswertund/oder Anzeigeeinheit (10) wird ein Signal in einer innerhalb des Störungsbereichs (S) angeordneten Aufnahmeanordnung (1) behandelt. Das EKG- Signal (E) wird zuerst analog überlinear komprimiert, anschliessend digitalisiert und als digitalisiertes Signal (D) an eine Auswert- und/oder Anzeigeeinheit (10) übermittelt. Das digitale Signal (D) wird in der Auswert- und/oder Anzeigeeinheit (10) digital überlinear expandiert.

FIG.1

**Beschreibung**

Die Erfindung betrifft ein Verfahren und eine Vorrichtung zum Erfassen und zum Übermitteln von elektrophysiologischen Signalen, insbesondere EKG-Daten sowie eine Vorrichtung zur Aufnahme einer Kernspintomographie (nachfolgend MRI-Bild) mit den Merkmalen des Oberbegriffs der unabhängigen Patentansprüche.

Elektrokardiogramme werden heute in einer Vielzahl zur Diagnose und Überwachung von Patienten eingesetzt. Die von Elektroden eines Elektrokardiographiegerätes aufgenommenen EKG-Signale sind zuerst Analogsignale. Die Signale werden in moderneren Geräten zur Datenbearbeitung und/oder Anzeige digitalisiert. Der Betrieb von solchen Geräten erfolgt häufig mittels Batterien oder Akkumulatoren, damit Geräte unabhängig von einem Stromnetz einsetzbar sind. Die Umwandlung des analogen Signals in ein digitales Signal in einem A/D Wandler ist bei Batterie-betriebenen Geräten problematisch, sobald verhältnismässig hohe Eingangsspannungen auftreten. Solche Spannungen können beispielsweise auch auf Grund von Störsignalen entstehen.

EKG's werden unter anderem zur Überwachung von Patienten bei der Aufnahme von Kernspintomographien eingesetzt. Ein Problem bei solchen Anwendungen besteht darin, dass durch das starke Magnetfeld und die hochfrequenten Anregungen des MRI im EKG-Gerät Störsignale, beispielsweise in den Elektroden, in den die Elektroden mit einer Auswerteinheit verbindenden elektrischen Leitern oder in der Auswerteinheit erzeugt werden.

Es sind verschiedene Wege vorgeschlagen worden, um diese Probleme zu lösen und um eine Überwachung von Patienten mittels EKG während der Aufnahme von MRI-Bildern zu ermöglichen. Ein weiteres Problem besteht darin, das aufgenommene EKG-Signal ohne Störungen aus dem MRI Tunnel hinaus zu einer Auswerteeinheit zu übermitteln.

In US 6 073 029 oder in US 6 032 063 sind beispielsweise spezielle Elektroden-Konstruktionen und Verkabelungen zur Aufnahme von diagnostischen EKGs während einer Aufnahme eines MRIs gezeigt.

In EP 1 050 270 ist ein Verfahren zum Bearbeiten von elektrokardiographischen Daten gezeigt, mittels welchem Störsignale eliminiert werden, welche durch einen MRI erzeugt werden.

In EP 695 139 ist ein EKG Sensor gezeigt, welcher zum Einsatz in einem MRI geeignet ist und der sich durch eine spezifische Elektrodenkonstruktion auszeichnet. In US 5 733 247 ist eine optische Verbindung zwischen einem Patientenmonitor und einem Kontrollpult gezeigt.

In WO92/21286 ist eine Vorrichtung zum Monitoring eines Patienten in einem MRI gezeigt. Um Interferenzen mit dem Magnetfeld des MRI zu vermeiden, werden Filter und Abschirmungen eingesetzt. In FR 2 685 968 ist vorgeschlagen, Störungen zwischen einem Kernspintomographen und einem Elektrokardiographiegerät, welches im Tomographen angeordnet ist, durch Verwendung von optischen Übertragungsmitteln zu eliminieren.

In US 4 991 580 ist ein Verfahren zur Datenbearbeitung von EKG Daten vorgeschlagen, welche an einem Patienten abgenommen werden, von welchem eine Kernspintomographie erstellt wird. Dazu werden entsprechende Filter und Verstärker eingesetzt.

In US 4 951 672 wird eine Elektrode zum Einsatz in Kernspintomographen mit spezifischen elektrischen Leitern vorgeschlagen.

Aus EP 132 785 ist zur Gewinnung eines EKG-Signals in einem Kernspintomographen bekannt, ein Verbindungskabel, welches zwischen Elektroden und dem EKG Verarbeitungsgerät angeordnet ist, mit einer Abschirmung zu versehen.

Aus US 6 052 614 ist ebenfalls ein Elektrokardiographiegerät gezeigt, welches in Kombination mit einer MRI Vorrichtung verwendet werden kann. Zur Übertragung der EKG Daten sind Filter und Verstärker sowie optische Übertragungsmittel vorgesehen.

Es ist eine Aufgabe der vorliegenden Erfindung, die Nachteile des Bekannten zu vermeiden, insbesondere also ein Verfahren und eine Vorrichtung zum Erfassen und Übermitteln von elektrophysiologischen Signalen wie z.B. EKG Daten zu schaffen, welches auch bei Batteriebetrieb eine Digitalisierung des erfassten Signals erlaubt. Eine weitere Aufgabe der Erfindung besteht darin, ein Verfahren und eine Vorrichtung zum Erfassen und Übertragen von Signalen zu schaffen, welche die Erfassung von Signalen gleichzeitig zur Aufnahme einer Kernspintomographie erlauben. Eine weitere Aufgabe der Erfindung besteht darin, eine MRI-Vorrichtung zu schaffen, welche das gleichzeitige Erfassen von elektrophysiologischen Signalen erlaubt.

Erfindungsgemäss werden diese Aufgaben mit einem Verfahren und mit einer Vorrichtung mit den Merkmalen des kennzeichnenden Teils der unabhängigen Patentansprüche gelöst.

Das erfindungsgemässe Verfahren lässt sich auf verschiedene Arten von elektrophysiologischen Signalen anwenden, beispielsweise Elektroencephalogramme oder Elektrokardiogramme. Im vorliegenden ist die Erfindung an Hand einer EKG Aufnahmeanordnung erläutert. Selbstverständlich lässt sich die Erfindung in ähnlicher Weise in Kombination mit anderen Vorrichtungen zum Messen von anderen elektrophysiologischen Signalen einsetzen.

Beim erfindungsgemässen Verfahren werden in einer EKG Aufnahmeanordnung EKG Daten erfasst. Dabei wird ein analoges EKG-Signal erzeugt. Das EKG-Signal wird anschliessend komprimiert. Dadurch wird ein komprimiertes Analogsignal erzeugt. Die Komprimierung erfolgt überlinear. Unter überlinear wird hier und im Folgenden eine Komprimierungsfunktion verstanden, bei der die Komprimierung stärker als linear erfolgt. Überlinear heisst also, dass der Grad der Komprimierung bei höheren Werten des Ausgangssignals höher ist als bei niedrigeren Werten des Ausgangssignals. Die Komprimierung kann typischerweise polynomial sein.

Das komprimierte Analogsignal wird anschliessend in ein Digitalsignal umgewandelt. Das Digitalsignal wird von der EKG Aufnahmeanordnung an eine Auswert- und/oder Anzeigeeinheit übermittelt. In der Auswert- und/oder Anzeigeeinheit wird das übermittelte Digitalsignal entsprechend der Komprimierungsfunktion wieder expandiert. Das expandierte Digitalsignal wird in der Auswert und/oder Anzeigeeinheit weiterbearbeitet oder angezeigt.

Es ist ausserdem möglich, dass Digitalsignal in der EKG Aufnahmeanordnung digital zu bearbeiten. Beispielsweise könnte das Signal digital gefiltert werden.

Durch die überlineare Komprimierung des analogen EKG-Signals können insbesondere Störsignale mit hohen Amplituden reduziert werden. Das komprimierte Analogsignal kann in einem A/D Wandler einfach in ein digitales Signal umgewandelt werden, ohne dass grosse Spannungen einer Spannungsquelle erforderlich sind.

Besonders einfach lässt sich das analoge EKG-Signal logarithmisch komprimieren und entsprechend das digitale Signal digital wieder exponentiell expandieren.

Besonders vorteilhaft lässt sich das erfindungsgemässe Verfahren zum Erfassen und Übermitteln von EKG Daten zeitgleich zur Aufnahme von MRI Bildern anwenden. Dabei ist die EKG Aufnahmeanordnung innerhalb eines elektromagnetischen Störungsbereichs der MRI Anordnung angeordnet. Die Auswert- und/oder Anzeigeeinheit ist ausserhalb dieses Störungsbereichs angeordnet. Durch die lokale Umwandlung der EKG Daten in ein digitales Signal können die EKG Daten einfach in digitalisierter Form aus dem MRI Tunnel an eine externe Auswerteinheit geschickt werden. Dank der erfindungsgemässen Vorrichtung ist es ausserdem möglich, die EKG Aufnahmeanordnung mit Batterien zu betreiben, so dass keine Stromzufuhr von externen Spannungs- oder Stromquellen notwendig ist.

Das erfindungsgemässe Verfahren lässt sich sowohl bei geschlossenen als auch bei offenen MRI Systemen einsetzen.

Gemäss einer bevorzugten Ausführungsform erfolgt die Komprimierung des EKG-Signals und vorzugsweise auch die Umwandlung des komprimierten Analogsignal in ein Digitalsignal in einer direkt auf einer Elektrode der EKG Aufnahmeanordnung angebrachten Recheneinheit. Damit können insbesondere Störsignale verhindert werden, welche in Verbindungsleitungen zwischen den Elektroden und Auswerteinheiten erzeugt werden könnten.

Gemäss einer weiteren bevorzugten Ausführungsform wird das digitale Signal optisch von der EKG Aufnahmeanordnung an die Anzeige- und/oder Auswerteinheit übermittelt. Die optische Übermittlung des digitalisierten Signals ist insbesondere wegen der Unanfälligkeit gegenüber der Erzeugung von Störsignalen vorteilhaft.

Es sind aber auch andere Übertragungen, insbesondere Übertragung mittels elektrischen Leitern, Funk oder auch mechanische Übertragungsmittel (beispielsweise pneumatisch) denkbar.

Das erfindungsgemässe Verfahren lässt sich insbesondere auch verwenden, um ein während der Aufnahme einer MRI's erfasstes elektrophysiologisches Signal, beispielsweise ein EKG Signal zum sogenannten Gating zu verwenden. Dies heisst, dass Informationen des EKG Signals zum Triggern oder Synchronisieren eines MRI's verwendet werden können. Dies ist insbesondere möglich, weil beispielsweise bei EKG Signalen die Bewegung des Herzens zeitabhängig genau bekannt ist. Das Signal lässt sich aber auch zum Monitoring von Patienten einsetzen.

Alternativ ist es auch denkbar, das digitalisierte Signal auf elektrischem Weg über eine abgeschirmte Verbindungsleitung oder elektromagnetisch über Funk, z.B. via Bluetooth zu übermitteln.

Die erfindungsgemässe Vorrichtung dient zum Erfassen und zum Übermitteln von elektrophysiologischen Signalen, insbesondere EKG Daten. Die Vorrichtung eignet sich insbesondere zur Durchführung eines der vorstehend aufgeführten Verfahren. Die Vorrichtung weist eine an sich herkömmliche Aufnahmeanordnung zum Erzeugen wenigstens eines elektrophysiologischen Signals, insbesondere eines EKG-Signals auf.

Die Vorrichtung enthält ausserdem eine Auswerte- und/oder Anzeigeeinheit. Die Auswert- und/oder Anzeigeeinheit dient zum Behandeln und/oder zum Anzeigen von EKG Daten. Die Vorrichtung ist mit Übermittlungsmitteln versehen, welche zum Übertragen von Daten von der EKG Aufnahmeanordnung zur Anzeige und/oder Auswerteeinheit dienen. Erfindungsgemäss weist die EKG Aufnahmeanordnung Mittel zum überlinearen Komprimieren des EKG-Signals und zum Bilden eines komprimierten Analogsignals auf. Mittels einem A/D Wandler lässt sich das komprimierte Analogsignal in ein digitales Signal umwandeln. In der Anzeige und/oder Auswerteinheit sind ausserdem Mittel zum digitalen Expandieren des digitalen Signals enthalten. Die digitale Expansion erfolgt entsprechend der vorgängigen Komprimierung überlinear. Die Expansionsfunktion ist eine Umkehrfunktion der vorhergehenden Komprimierungsfunktion.

Bevorzugt sind die Mittel zum Komprimieren des EKG-Signals zum logarithmischen Komprimieren des EKG-Signals und die Mittel zum digitalen Expandieren zum exponentiellen Expandieren des digitalen Signals ausgebildet.

Gemäss einem bevorzugten Ausführungsbeispiel sind die Mittel zum Komprimieren und vorzugsweise auch der A/D Wandler durch eine Rechneranordnung gebildet, welche direkt auf einer Elektrode der EKG Aufnahmeanordnung angebracht ist. Es ist auch denkbar, eine entsprechende Rechneranordnung auf mehreren Elektroden vorzusehen.

Mit der erfindungsgemässen Vorrichtung lässt sich der Energieverbrauch reduzieren, da die Eingangssignale in den A/D-Wandler geringere Spannungen aufweisen.

Weiter vorzugsweise werden optische Übertragsungsmittel zum Übertragen des digitalisierten Signals von der EKG Aufnahmeanordnung zu der Anzeige- und Auswerteeinheit übertragen. Es sind aber auch andere, insbesondere elektrische Übermittlungen, insbesondere mittels einem abgeschirmten Kabel oder Funk, denkbar.

Grundsätzlich sind auch mechanische Übermittlungen, beispielsweise eine pneumatische Übermittlung des Signals denkbar und möglich.

Die erfindungsgemässe Vorrichtung zur Aufnahme eines MRIs ist im wesentlichen herkömmlich ausgebildet. Die Vorrichtung ist ausserdem mit einer wie vorstehend beschriebenen Vorrichtung zum Erfassen von elektrophysiologischen Daten ausgebildet.

Die Erfindung wird im Folgenden in Ausführungsbeispielen und an Hand der Zeichnungen näher erläutert. Es zeigen:

Figur 1: eine schematische Darstellung einer erfindungsgemässen Vorrichtung zum gleichzeitigen Aufnehmen einer Kernspintomographie und eines EKGs,

Figur 2: eine schematische Darstellung von einzelnen Bestandteile der erfindungsgemässen Vorrichtung,

Figur 3a: eine schematische Darstellung einer ersten Ausführungsform von Übertragungsmitteln,

Figur 3b: eine schematische Darstellung einer zweiten Ausführungsform von Übertragungsmitteln und

Figur 4a, 4b: schematische Darstellungen von mit Elektroden verbundenen Rechneranordnungen und

Figur 5a, 5b: eine schematische Darstellung eines unkomprimierten und eines komprimierten EKG-Signals mit Störungen.

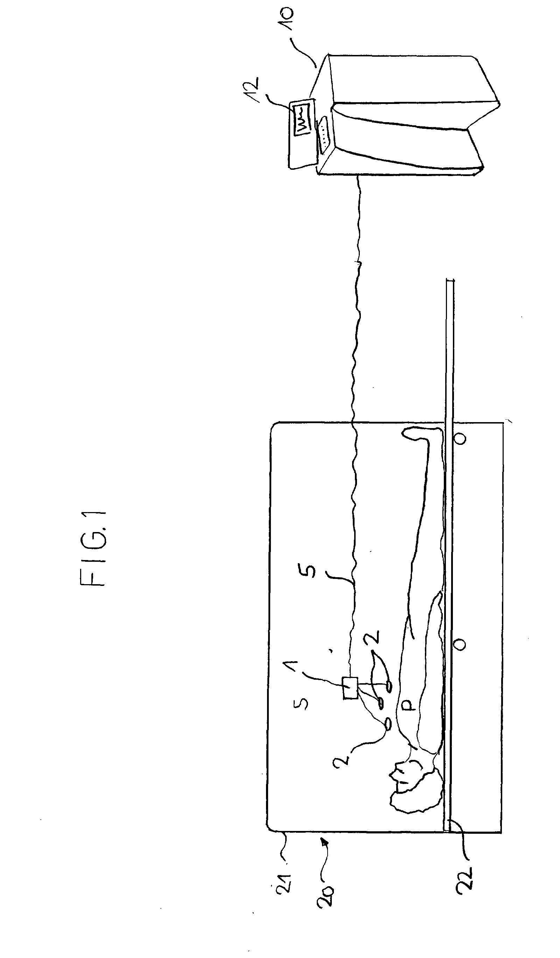

Figur 1 zeigt eine MRI Anordnung 20. Die Anordnung 20 weist in herkömmlicher Weise nicht näher dargestellte Bauelemente wie Magnetspule, Sender und Empfänger und entsprechende Detektionsmittel zum Erstellen von Kernspintomographien auf. Die MRI Anordnung 20 weist einen Tunnel auf. Die erfindungsgemässe Vorrichtung und das erfindungsgemässe Verfahren lassen sich aber auch im Zusammenhang mit offenen MRI Systemen einsetzen. Zur Diagnose kann ein Patient P auf einem verschiebbar gelagerten Bett 22 in den Tunnel hineingeschoben werden. Die MRI Anordnung 20 ist durch eine Abschirmung 21, welche einen Faradaykäfig bildet, gegen elektromagnetische Wellen abgeschirmt. Die spezifische Konstruktion der MRI Anordnung 20 ist nicht Gegenstand der vorliegenden Erfindung. Sie wird nicht genauer beschrieben.

Die MRI Anordnung 20 weist eine EKG Aufnahmeanordnung 1 auf. Die EKG Aufnahmeanordnung 1 ist im Ausführungsbeispiel mit drei Elektroden 2 versehen. Es können auch mehr oder weniger Elektroden eingesetzt werden. Die EKG Aufnahmeanordnung 1 weist in herkömmlicher Weise entsprechende Eingänge für die Elektroden, einen Mikroprozessor zum Bearbeiten der ermittelten EKG-Signale und eine Stromversorgung auf. Die EKG Aufnahmeanordnung 1 ist ausserdem mit einem Ausgabeanschluss zur Übermittlung von Daten versehen. In Figur 1 ist zur Übermittlung der Daten ein abgeschirmtes Kabel 5 gezeigt, welches EKG Daten an eine ausserhalb der MRI Anordnung 20 liegende Auswert- und/oder Anzeigeeinheit 10 übermittelt. Die Auswert- und/oder Anzeigeeinheit 10 ist mit einer Anzeige 12 zum Anzeigen von EKG Daten versehen. Die Auswert- und/oder Anzeigeeinheit 10 befindet sich ausserhalb eines Störungsbereichs S der MRI Anordnung. Die EKG Aufnahmeanordnung 1 befindet sich im durch die Abschirmung 21 begrenzten elektromagnetischen Störungsbereich S.

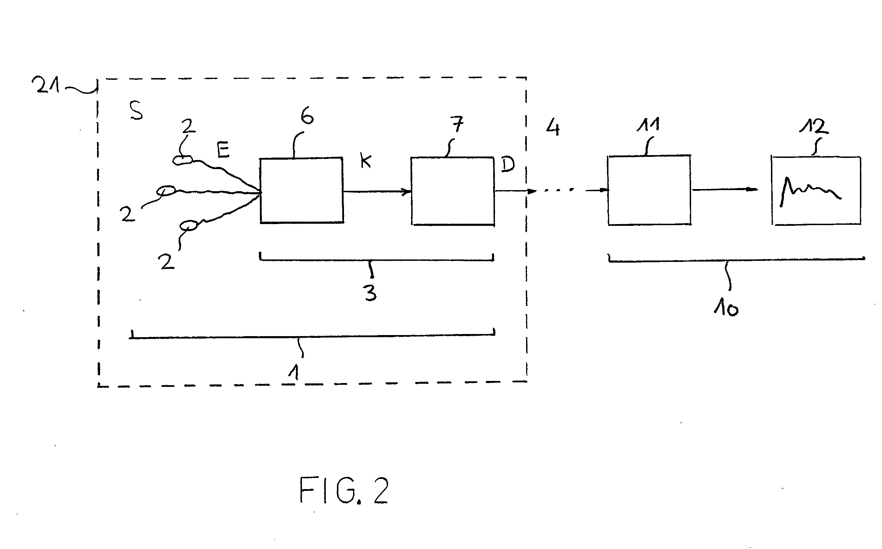

Figur 2 zeigt schematisch einzelne Komponenten der erfindungsgemässen Vorrichtung. Innerhalb der Abschirmung 21 befindet sich neben nicht im Detail dargestellten Elementen der MRI Anordnung 20 eine EKG Aufnahmeanordnung 1. Die EKG Aufnahmeanordnung 1 weist drei Elektroden 2 auf, mittels welchen ein analoges EKG-Signal E erzeugbar ist. Die Elektroden 2 sind über nicht genauer bezeichnete Kabel mit einer Rechnereinheit 3 verbunden. Zuerst wird das analoge EKG-Signal E in Komprimiermitteln 6 zu einem komprimierten analogen Signal K komprimiert. Das komprimierte Analogsignal K wird in einem A/D Wandler 7 zu einem Digitalsignal D digitalisiert. Die Komprimiermittel 6 werden durch einen herkömmlichen Komprimierer, z. B. durch einen Logarithmierer gebildet.

Als A/D Wandler wird beispielsweise ein herkömmlicher 16bit low power A/D Wandler eingesetzt.

Ausserdem ist es denkbar, in einem Signalprozessor zusätzliche Bearbeitungen des digitalen Signals vorzunehmen, beispielsweise das Signal zu filtern.

Das digitale Signal D wird im Ausführungsbeispiel gemäss Figur 2 über optische Übertragungsmittel 4 aus der Abschirmung 21 zu der Auswert- und/oder Anzeigeeinheit 10 übermittelt. In der Auswertund/oder Anzeigeeinheit 10 sind Mittel 11 zum digitalen Expandieren des digitalen Signals D enthalten. Die Mittel zum Expandieren sind beispielsweise durch einen entsprechend programmierten Mikroprozessor gebildet. Das expandierte Digitalsignal wird in der Auswert- und/oder Anzeigeeinheit 10 in an sich bekannter Weise bearbeitet und auf einer Anzeige 12 angezeigt. Das expandierte digitale Signal kann auf herkömmliche Weise bearbeitet werden, um Störungen durch die MRI Anordnung 20 zu eliminieren. Dazu kann beispielsweise eine digitale Filterung oder Template Matching vorgenommen werden.

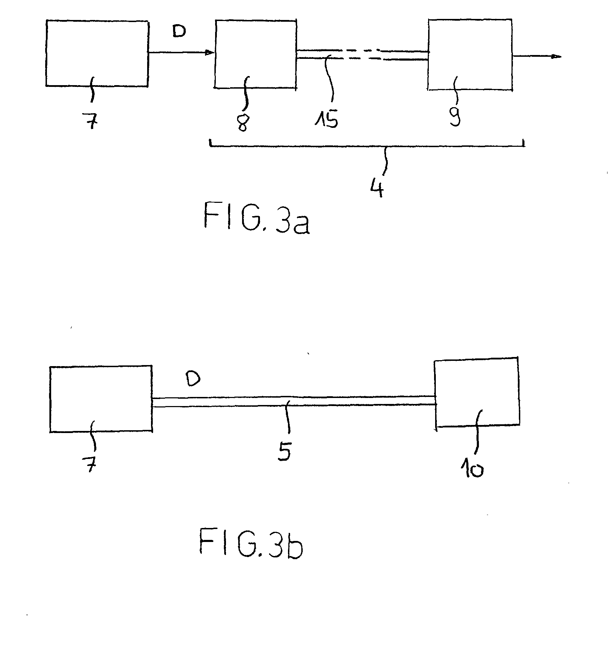

Figur 3a zeigt schematisch eine mögliche Ausführungsform von optischen Übertragungsmitteln. Das im A/D Wandler digitalisierte Signal D wird über eine optische Sendeanordnung 8, beispielsweise eine Laserdiode mit entsprechender Datenaufbereitung über einen Lichtwellenleiter 15 zu einem optischen Empfänger 9, typischerweise einem Fotodetektor gesendet, welcher das optische Signal in ein elektrisches Signal zurückwandelt und an die Mittel zum Expandieren 11 weitergibt.

In Figur 3b ist eine Alternative mit einem abgeschirmten Kabel 5 zur Übertragung des digitalen Signals D gezeigt.

Die innerhalb des Störungsbereichs 2 angeordneten Komponenten der erfindungsgemässen Vorrichtung, insbesondere die Komprimiermittel, der A/D Wandler und allfällige Komponenten von optischen Übertragungsmitteln sind in an sich bekannter Weise gegen elektromagnetische Störungen abgeschirmt. Diese Komponenten können in einem abgeschirmten Gehäuse angeordnet sein. Um Störungen in den Verbindungsleitungen zwischen den Elektroden 2 und der Rechnereinheit 3 zu verhindern, ist es gemäss der Erfindung ausserdem auch denkbar, die Rechnereinheit 3 direkt auf einer oder mehreren Elektroden anzuordnen.

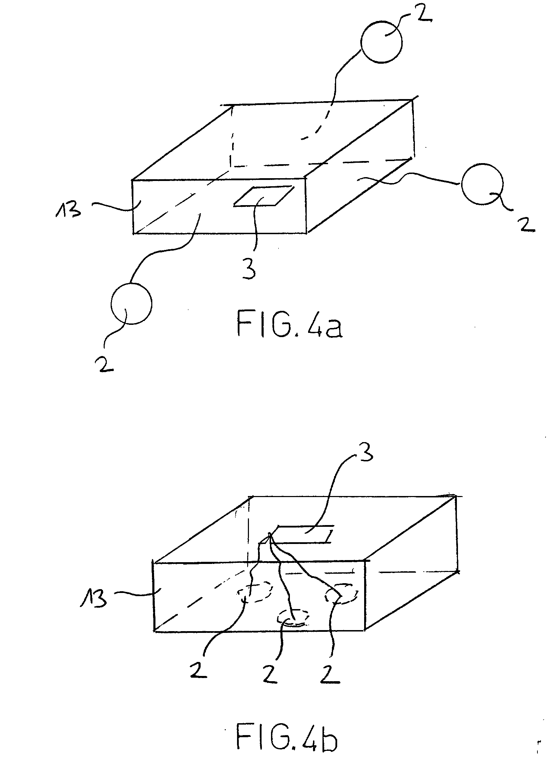

Figur 4a zeigt ein erstes Ausführungsbeispiel einer erfindungsgemässen Vorrichtung. Die Rechnereinheit 3 ist in einem Gehäuse 13 angeordnet. Am Gehäuse 13 sind über kurze elektrische Verbindungen drei Elektroden 2 vorgesehen. Das Gehäuse 13 ist beispielsweise aus Kupfer oder aus anderem antimagnetischen Material gebildet. Typischerweise kann das Gehäuse eine Grösse von einigen Zentimetern aufweisen.

Figur 4b zeigt eine alternative Ausführungsform. In der Ausführung gemäss Figur 4b ist die Rechnereinheit 3 ebenfalls in einem Gehäuse 13, vorzugsweise aus Kupfer angeordnet. Die Elektroden 2 sind direkt im Gehäuse 13 integriert. Auf diese Weise wird sichergestellt, dass auch in den Verbindungsleitungen zwischen Elektroden 2 und der Rechnereinheit 3 keine Störungen auftreten.

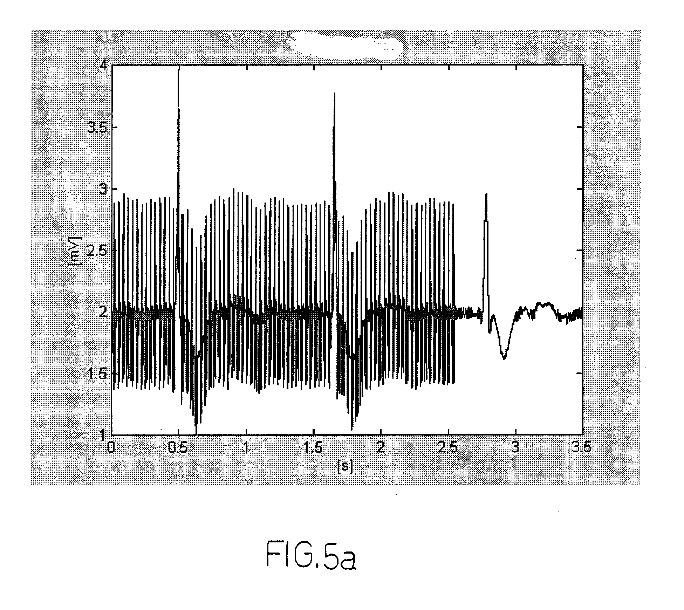

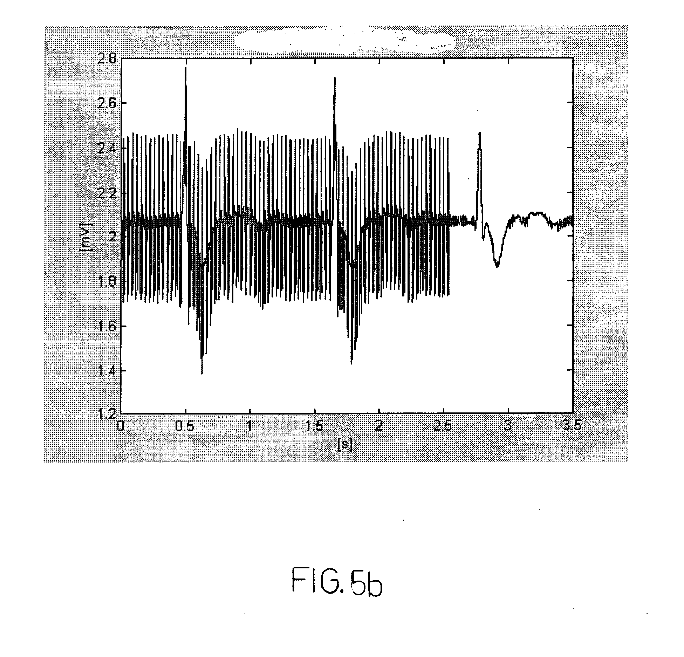

Figuren 5a und 5b zeigen typische EKG-Signale und deren Bearbeitung.

In Figur 5a ist ein herkömmliches analoges EKG-Signal gezeigt. Im EKG-Signal sind durch eine MRI Anordnung erzeugte Störungen gezeigt.

In Figur 5b ist das logarithmisch komprimierte Analogsignal K dargestellt. Die Komprimierung erfolgt gemäss der Formel

$$K = a \times \log (b \times E).$$

Die Faktoren a und b können geeignet gewählt werden, wobei je nach Ausgangssituation durch Experimentieren die optimalen Faktoren auffindbar sind. Die Berechnung erfolgt mit einem natürlichen Logarithmus.

Das unkomprimierte Signal wurde unter Einfluss eines starken, elektromagnetischen Gradientenfeldes aufgenommen. Zwischen dem Zeitpunkt 0 und 2,5 Sekunden ist das Magnetfeld eingeschaltet. Das Magnetfeld führt zu einer Störung von ca. 3 mV. Diese Störung ist dem QRS-Komplex überlagert, sodass sich Maxima von bis gegen 4 mV ergeben. Diese Amplitude ist für eine Signalverarbeitung mit den gewünschten Geräten, insbesondere einem operationellen Verstärker und einem A/D Wandler zu gross.

In Figur 4b ist das Signal nach Kompression gezeigt. Die Störung wurde auf 2,4 mV reduziert. Die Amplitude des maximalen Signals beträgt dank der logarithmischen Kompression nur noch 2,8 mV. Dieses Signal lässt sich problemlos weiter verarbeiten.

Das digitale Signal D wird in der Expandiereinheit wie folgt expandiert:

$$D_e = 1/b \times e^{(D/a)}$$

**Patentansprüche**

1.  Verfahren zum Erfassen von elektrophysiologischen Daten, insbesondere von EKG Daten in einer Aufnahmeanordnung (1) und zum Übermitteln der Daten in eine Auswert- und/oder Anzeigeeinheit (10), bestehend aus den Schritten

    -    Erfassen eines Signals (E), insbesondere eines EKG Signals in der Aufnahmeanordnung (1)
    -    Überlineares Komprimieren des Signals (E) zum Bilden eines komprimierten Analogsignals (K)
    -    Umwandeln des komprimierten Analogsignals (K) in ein Digitalsignal (D)
    -    Übermitteln des Digitalsignals (D) an die Auswertund/oder Anzeigeeinheit (10)
    -    digitales Expandieren des Digitalsignals (D)
    -    Anzeigen und/oder Bearbeiten des expandierten Digitalsignals ($D_e$).

2.  Verfahren nach Anspruch 1, **dadurch gekennzeichnet, dass** das Signal (E) logarithmisch komprimiert wird.

3.  Verfahren nach einem der Ansprüche 1 oder 2, **dadurch gekennzeichnet, dass** die Erfassung und Übermittlung der elektrophysiologischen Daten zeitgleich zur Vornahme einer MRI Diagnose an einem Patienten (P) vorgenommen wird, wobei sich die Aufnahmeanordnung (1) innerhalb und die Auswertund/oder Anzeigeeinheit (10) ausserhalb eines elektromagnetischen Störungsbereichs (S) einer MRI Anordnung (20) zur Vornahme der Diagnose befindet.

4. Verfahren nach einem der Ansprüche 1 bis 3, **dadurch gekennzeichnet, dass** die Komprimierung des Signals (E) und vorzugsweise die Umwandlung des Analogsignals (K) in das Digitalsignal (D) in einer direkt auf einer Elektrode (2) der Aufnahmeanordnung (1) angebrachten Recheneinheit (3) erfolgt.

5. Verfahren nach einem der Ansprüche 1 bis 4, **dadurch gekennzeichnet, dass** das digitale Signal (D) optisch an die Auswert- und/oder Anzeigeeinheit (10) übermittelt wird.

6. Verfahren nach einem der Ansprüche 1 bis 4, **dadurch gekennzeichnet, dass** das digitale Signal (D) elektrisch über eine abgeschirmte Verbindungsleitung (5) oder drahtlos über Funk an die Auswertund/oder Anzeigeeinheit (10) übermittelt wird.

7. Vorrichtung zum Erfassen und Übermitteln von elektrophysiologischen Daten, insbesondere zum Durchführen eines Verfahrens nach einem der Ansprüche 1 bis 6,
mit einer Aufnahmeanordnung (1) zum Erzeugen wenigstens eines Signals (E) und mit einer
Auswert- und/oder Anzeigeeinheit (10) zum Behandeln und/oder Anzeigen von elektrophysiologischen Daten
und mit Übertragungsmitteln (4; 5) zum Übertragen von Daten von der Aufnahmeanordnung (1) zur Auswert- und/oder Anzeigeeinheit (10),
**dadurch gekennzeichnet, dass** die Aufnahmeanordnung (1) Mittel (6) zum überlinearen Komprimieren des Signals (E) zum Bilden eines komprimierten Analogsignals (K) und einen A/D Wandler (7) zum Umwandeln des komprimierten Analogsignals (K) in ein digitales Signal (D) aufweist
und dass die Auswert- und/oder Anzeigeeinheit (10) Mittel (11) zum digitalen Expandieren des digitalen Signals (D) aufweist.

8. Vorrichtung nach Anspruch 7, **dadurch gekennzeichnet, dass** die Mittel (6) zum Komprimieren des Signals (E) zum logarithmischen Komprimieren des Signals ausgebildet sind.

9. Vorrichtung nach einem der Ansprüche 7 oder 8, **dadurch gekennzeichnet, dass** die Mittel (6) zum Komprimieren und vorzugsweise der A/D Wandler (7) durch eine Rechneranordnung (3) gebildet sind, welche direkt auf einer Elektrode (2) der Aufnahmeanordnung (1) angeordnet sind.

10. Vorrichtung nach einem der Ansprüche 7 bis 9, **dadurch gekennzeichnet, dass** die Übertragungsmittel optische Übertragungsmittel (4) sind oder durch ein abgeschirmtes Kabel (5) gebildet sind.

11. Vorrichtung zur Vornahme einer MRI Diagnose mit einer Vorrichtung zum gleichzeitigen Erfassen von elektrophysiologischen Daten nach einem der Ansprüche 7 bis 10.

FIG.1

FIG.2

FIG.3a

FIG.3b

FIG. 4a

FIG.4b

FIG.5a

FIG.5b

**Europäisches Patentamt**

## EUROPÄISCHER RECHERCHENBERICHT

Nummer der Anmeldung

EP 02 40 5968

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| Y | US 6 148 229 A (MORRIS SR. ET AL.) 14. November 2000 (2000-11-14) * Spalte 7, Zeile 51 - Spalte 8, Zeile 19; Abbildungen 1-3 * --- | 1-3,5-8, 10,11 | A61B5/0428 A61B5/055 A61B5/024 |
| Y | EP 0 913 703 A (MATSUSHITA ELECTRIC INDUSTRIAL CO. LTD.) 6. Mai 1999 (1999-05-06) * Zusammenfassung; Abbildungen 1-6 * --- | 1-3,5-8, 10,11 | |
| A | US 6 045 504 A (MUZILLA ET AL.) 4. April 2000 (2000-04-04) * Zusammenfassung; Abbildungen 1-8 * --- | 1,2,7,8 | |
| A | US 5 464 014 A (SUGAHARA) 7. November 1995 (1995-11-07) * Zusammenfassung; Abbildungen 1-7 * --- | 1,6,7,10 | |
| A | US 5 436 564 A (KREGER ET AL.) 25. Juli 1995 (1995-07-25) * das ganze Dokument * ----- | 1,7 | |

RECHERCHIERTE SACHGEBIETE (Int.Cl.7)

A61B

Der vorliegende Recherchenbericht wurde für alle Patentansprüche erstellt

| Recherchenort | Abschlußdatum der Recherche | Prüfer |
|---|---|---|
| DEN HAAG | 22. April 2003 | Hunt, B |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

&: Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**  EP 02 40 5968

In diesem Anhang sind die Mitglieder der Patentfamilien der im obengenannten europäischen Recherchenbericht angeführten
Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

22-04-2003

| Im Recherchenbericht angeführtes Patentdokument | | Datum der Veröffentlichung | Mitglied(er) der Patentfamilie | | Datum der Veröffentlichung |
|---|---|---|---|---|---|
| US 6148229 | A | 14-11-2000 | KEINE | | |
| EP 913703 | A | 06-05-1999 | JP | 2814900 B2 | 27-10-1998 |
| | | | JP | 7155321 A | 20-06-1995 |
| | | | EP | 0913703 A1 | 06-05-1999 |
| | | | CA | 2137430 A1 | 08-06-1995 |
| | | | DE | 69419237 D1 | 29-07-1999 |
| | | | DE | 69419237 T2 | 25-11-1999 |
| | | | DE | 69431197 D1 | 19-09-2002 |
| | | | EP | 0657747 A1 | 14-06-1995 |
| | | | US | 5507293 A | 16-04-1996 |
| US 6045504 | A | 04-04-2000 | KEINE | | |
| US 5464014 | A | 07-11-1995 | JP | 1916836 C | 23-03-1995 |
| | | | JP | 5115459 A | 14-05-1993 |
| | | | JP | 6016772 B | 09-03-1994 |
| | | | JP | 6269426 A | 27-09-1994 |
| | | | JP | 8008919 B | 31-01-1996 |
| | | | US | 5239265 A | 24-08-1993 |
| US 5436564 | A | 25-07-1995 | CA | 2042271 A1 | 07-02-1992 |
| | | | EP | 0470764 A2 | 12-02-1992 |
| | | | IL | 98926 A | 28-11-1994 |
| | | | JP | 1917503 C | 23-03-1995 |
| | | | JP | 4244136 A | 01-09-1992 |
| | | | JP | 6016771 B | 09-03-1994 |

EPO FORM P0461

Für nähere Einzelheiten zu diesem Anhang : siehe Amtsblatt des Europäischen Patentamts, Nr.12/82